# FASCICULE DE BREVET EUROPEEN

(11) **EP 1 706 017 B1**
(45) Date de publication et mention de la délivrance du brevet: **27.11.2013**
(21) Numéro de dépôt: 05717462.5
(22) Date de dépôt: 21.01.2005
(51) Int. Cl.: A61B 3/00

(54) **APPAREIL D'EXAMEN DE L'OEIL PAR TOMOGRAPHIE AVEC DISPOSITIF DE VISEE**
TOMOGRAFIE-AUGENUNTERSUCHUNGSGERÄT MIT VISIERVORRICHTUNG
EYE EXAMINATION DEVICE BY MEANS OF TOMOGRAPHY WITH A SIGHTING DEVICE

(30) Priorité: 22.01.2004 FR 0400581
(43) Date de publication de la demande: 04.10.2006
(73) Titulaire: CENTRE NATIONAL DE LA RECHERCHE SCIENTIFIQUE (CNRS), 75794 Paris Cedex 16 (FR); Observatoire de Paris, 75014 Paris (FR); Mauna Kea Technologies, 75010 Paris (FR)
(72) Inventeur: LACOMBE, François, F-92370 Chaville (FR); LAFAILLE, David, 76600 Le Havre (FR); GLANC, Marie, F-91190 Meudon (FR); GENDRON, Eric, F-92190 Meudon (FR); STEFANOVITCH, Douchane, F-92190 Meudon (FR)
(74) Mandataire: Pontet Allano & Associes
(86) Numéro de dépôt international: PCT/FR2005/000133
(87) Numéro de publication internationale: WO 2005/079655

(56) Documents cités:
- US-A- 3 836 238
- US-A- 5 565 949

## Description

La présente invention concerne un dispositif de visée pour un examen de l'oeil. Elle vise également un procédé de visée mis en oeuvre dans ce dispositif, ainsi qu'un système d'examen de l'oeil par tomographie in vivo équipé de ce dispositif.

Lors de l'examen de l'oeil en général, et en particulier de la rétine, les mouvements inconscients de l'oeil, même pendant une fixation, peuvent considérablement limiter les performances de l'examen.

Les mouvements résiduels pendant une fixation sont de trois types :
- Nystagmus physiologique (ou tremor) : oscillations très rapides (de 40 à 100 Hz), de petite amplitude (déplacement des images de l'ordre du micron sur la rétine) ;
- Dérives : mouvements lents (1 µm en quelques ms), décorrélés d'un oeil à l'autre ;
- Micro-saccades : mouvements très rapides (quelques centaines par seconde), corrélés entre les yeux, de recentrage approximatif du champ.

L'expérience montre que les performances de fixation d'un sujet donné sont très variables, suivant son état de fatigue, suivant l'éclairage ambiant, ou suivant la durée de la fixation. Il est par ailleurs connu que la fixation avec deux yeux est plus performante qu'avec un seul.

US 3 836 238 et US 5 565 949 divulguent des dispositifs d'affichage de cibles de fixation pour un examen de l'oeil d'un sujet. Des intervalles de fixation sont alternés avec des intervalles de repos.

Le dispositif de US 3 836 238 est divulgué en combinaison avec un aberromètre, alors que le dispositif de US 5 565 949 est destiné à effectuer des mesures de champ visuel.

L'adjonction d'un système de compensation des mouvements de l'oeil peut se révéler très complexe, coûteux, et parfois incompatible avec l'instrumentation existante.

Le but de la présente invention est de remédier à ces inconvénients en proposant un dispositif de visée qui optimise la performance de fixation du sujet, ce dispositif de visée étant destiné à équiper un système d'examen en lui procurant une très bonne résolution spatiale. Il s'agit donc d'augmenter la performance globale de l'examen en augmentant celle du sujet.

Suivant l'invention, le dispositif de visée comprend au moins une cible mobile présentant une forme et une trajectoire programmables, cette ou ces cibles étant affichée(s) sur des moyens de visualisation tels qu'un écran et visible des deux yeux, pendant la durée de l'examen.

Dans un premier mode opératoire, la ou les cibles sont déplacées de manière à alterner des intervalles de fixation sur une position donnée avec des intervalles dits de repos sur une ou plusieurs autres positions. La durée des intervalles de fixation peut être ajustée pour en optimiser la qualité. On peut également ajuster la diversité, la position et la durée des positions de repos.

Dans un second mode opératoire, on commande un mouvement continu qui forcerait le regard du sujet à suivre une cible mobile. Si les performances de suivi sont meilleures que celles de fixation, la connaissance a priori de la trajectoire permettrait de recaler les images de l'oeil obtenues avec une meilleure précision qu'elles ne le sont quand on observe une cible immobile.

Suivant un autre aspect de l'invention, il est proposé un système d'examen de l'oeil par tomographie in vivo comprenant :
- un interféromètre de Michelson, réalisant un montage d'OCT plein champ,
- un dispositif d'optique adaptative, disposé entre l'interféromètre et un oeil à examiner, réalisant la correction des fronts d'onde en provenance de l'oeil mais aussi à destination de l'oeil, et
- un dispositif de détection, disposé en aval de l'interféromètre, permettant sans modulation ni détection synchrone, de réaliser la mesure interférométrique selon le principe de l'OCT,
caractérisé en ce qu'il comprend en outre un dispositif de visée selon l'invention, comprenant au moins une cible mobile présentant une forme et une trajectoire programmable, ladite cible étant affichée sur des moyens de visualisation et visible d'au moins un des yeux dudit patient, pendant la durée de l'examen.

Ce dispositif de visée permet de guider le regard du patient tout en assurant son confort visuel et en optimisant ses performances de fixation.

D'autres avantages et caractéristiques de l'invention apparaîtront à l'examen de la description détaillée d'un mode de mise en oeuvre nullement limitatif, et des figures annexées dans lesquelles :
- la figure 1 illustre la structure d'un système de tomographie in vivo intégrant un dispositif de visée selon l'invention, et
- les figures 2A et 2B représentent respectivement un premier et un second exemples de réalisation de mires actives mises en oeuvre dans un dispositif de visée selon l'invention, sur un écran d'un ordinateur.
- la figure 3 est un schéma d'un autre exemple de réalisation d'un système de tomographie in vivo selon l'invention.

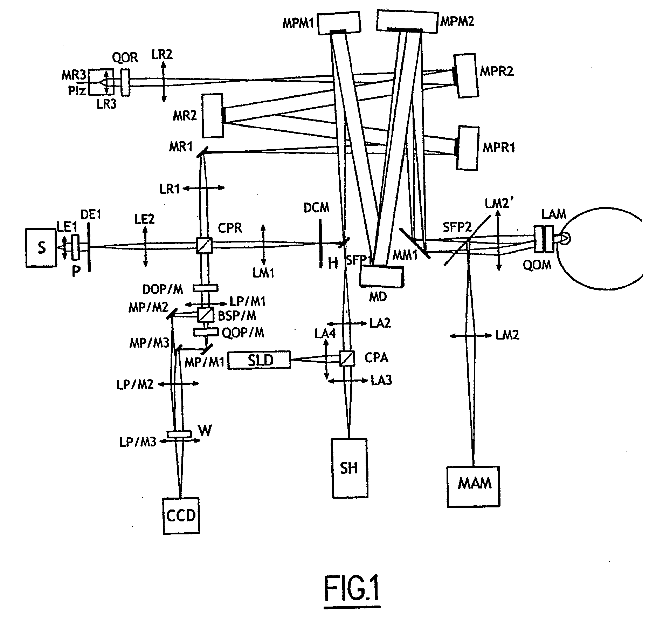

On va maintenant décrire, en référence à la figure 1, un exemple pratique de réalisation d'un système de tomographie in vivo selon l'invention. Ce système comprend un interféromètre, de type Michelson, comportant un bras de mesure prévu pour illuminer l'oeil et collecter la lumière renvoyée, et un bras de référence prévu pour illuminer un miroir mobile permettant l'exploration en profondeur du tissu rétinien.

L'interféromètre est utilisé en lumière polarisée de façon rectiligne et perpendiculaire dans les deux bras. La source de lumière S est une diode à faible longueur de cohérence temporelle (par exemple, 12 µm), dont le spectre est centré sur 780 nm. Elle confère par principe au système de tomographie in vivo une résolution axiale égale à la moitié de la longueur de cohérence divisée par l'indice de réfraction du milieu.

Cette source de lumière S peut être pulsée. Dans ce cas, elle est alors synchronisée avec la prise d'image et la correction adaptative. Le faisceau est limité par un diaphragme de champ correspondant à 1 degré dans le champ de vue de l'oeil (300 µm sur la rétine) et un diaphragme pupillaire correspondant à une ouverture de 7 mm sur un oeil dilaté.

Un polariseur d'entrée P permet l'équilibrage optimal des flux injectés dans les deux bras de l'interféromètre.

Les deux bras présentent une configuration dite de Gauss, afocale, qui permet le transport des pupilles, d'une part, et la matérialisation d'une image intermédiaire du champ où un diaphragme bloque une grande part du reflet cornéen, d'autre part. Des lames quart d'onde assurent par la rotation de la polarisation de la seule lumière renvoyée par l'oeil, et le miroir mobile, un filtrage efficace des réflexions parasités dans le système de tomographie in vivo selon l'invention.

Afin de conserver l'égalité des chemins optiques dans les deux bras, avec le même transport des pupilles et du champ, le bras de référence est similaire au bras de mesure, mais avec une optique statique.

On va maintenant décrire la voie de détection du système de tomographie in vivo selon l'invention. Les deux faisceaux sur le bras de sortie sont encore polarisés perpendiculairement, et ils n'interfèrent que s'ils sont projetés sur une direction commune. Un prisme de Wollaston W a pour fonction de projeter simultanément les deux rayonnements sur deux directions d'analyse perpendiculaires. On peut alors effectuer une mesure simultanée de l'intensité après interférence dans deux états d'interférence en opposition, sans modulation ni détection synchrone, sur un détecteur bidimensionnel unique. L'adjonction d'une lame quart d'onde, après division du faisceau, permet d'accéder à deux mesures supplémentaires, levant ainsi toute ambiguïté entre amplitude et phase des franges. Une lame demi onde à l'entrée de la voie de détection permet d'orienter convenablement les polarisations incidentes.

Le prisme de Wollaston est placé dans un plan pupillaire, donc conjugué du cube séparateur de l'interféromètre de Michelson. L'angle de séparation du prisme de Wollaston est choisi en fonction du champ à observer. La longueur focale de l'objectif final détermine le pas d'échantillonnage des quatre images.

Le détecteur est du type CCD, avec une cadence d'image supérieure à 30 images par seconde. Ce détecteur est associé à un calculateur dédié (non représenté) dans lequel est réalisé le traitement numérique des images : extraction des quatre mesures, étalonnage, calcul de l'amplitude des franges.

La correction adaptative des fronts d'onde est réalisée en amont de l'interféromètre, donc dans le bras de mesure. Chaque point de la source S voit ainsi son image sur la rétine corrigée des aberrations, et l'image en retour est également corrigée. L'amplitude des franges est alors maximale.

Le sous-ensemble d'optique adaptative comprend un miroir déformable MD. La mesure de front d'onde est faite par un analyseur SH de type Shack-Hartmann sur le faisceau de retour d'un spot lumineux lui-même imagé sur la rétine via le miroir déformable MD. La longueur d'onde d'analyse est de 820 nm. L'éclairage est continu et fourni par une diode SLD superluminescente temporellement incohérente. Le dimensionnement de l'analyseur correspond à une optimisation entre sensibilité photométrique et échantillonnage du front d'onde. La cadence de rafraîchissement de la commande du miroir déformable MD peut atteindre 150 Hz. Un calculateur dédié (non représenté) gère la boucle d'optique adaptative. La commande est toutefois synchronisée pour geler la forme du miroir pendant la mesure interférométrique.

Un contrôle approprié de la focalisation de la voie d'analyse, au moyen d'une lentille LA2, permet d'adapter la distance de focalisation à la couche sélectionnée par l'interféromètre. Cette disposition est capitale pour conserver un contraste optimal à toute profondeur.

Le miroir déformable MD est conjugué de la pupille du système et de l'oeil. Le champ du système est défini par le diaphragme de champ DCM d'entrée du système. Il est de préférence choisi à une valeur inférieure à celle du champ d'isoplanétisme de l'oeil, ce qui garantit la validité de la correction adaptative dans le champ sur la seule mesure de front d'onde réalisée à partir du spot, au centre du champ. A titre d'exemple, le champ du système peut être choisi égal à 1 degré, mais la valeur de ce champ pourrait être augmentée.

De plus, la rotation du miroir déformable MD permet de choisir l'angle d'arrivée du faisceau dans l'oeil, donc la portion de rétine étudiée.

L'adjonction de verres correcteurs de la vue du sujet, donc des bas ordres d'aberrations géométriques tels que le focus ou l'astigmatisme, juste devant l'oeil, permet de relâcher les exigences sur la course du miroir déformable MD, et garantit également une meilleure visée. Un système correcteur adaptatif par transmission peut être utilisé de préférence à des verres fixes pour une correction optimale.

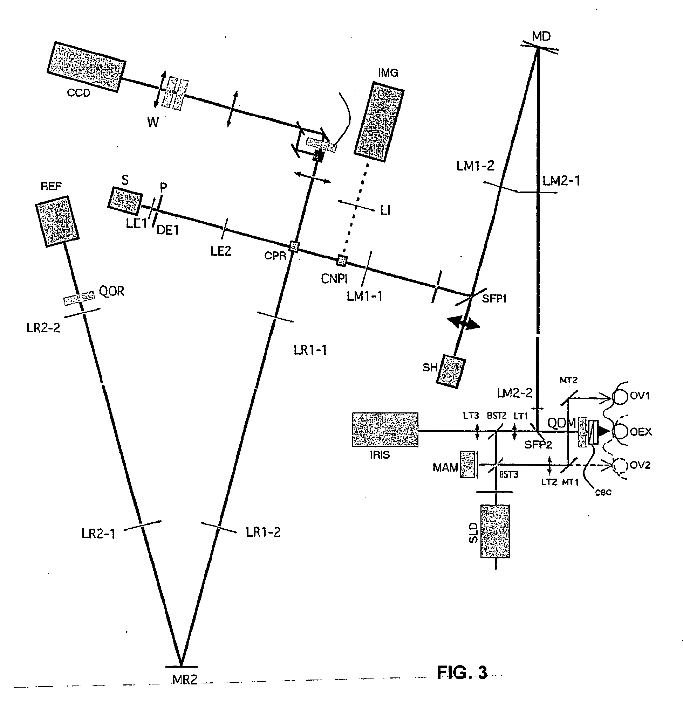

Ainsi qu'illustré en figure 3, le système peut en outre comprendre des moyens d'imagerie classique, comme une caméra IMG, permettant d'associer les mesures interférométriques avec une imagerie simple des zones examinées, par exemple pour faciliter l'exploration et la sélection des zones à examiner.

Placé directement en sortie (au retour) du bras de mesure, donc juste avant le cube polarisant CPR de l'interféromètre, un second cube polarisant CNPI permet de dévier le faisceau de retour vers une caméra d'imagerie IMG disposant de ses propres moyens de focalisation LI de l'image. Sur cette voie, une image directe de la zone rétinienne visée sera observable. On peut en particulier agencer le bras de mesure et cette voie additionnelle de sorte qu'ils procurent un champ d'observation plus large que le mode interférométrique, dont le champ est limité en particulier par la technique de mesure de contraste interférométrique en elle-même.

Un dispositif de visée selon l'invention, collaboratif ou actif, est installé en amont de l'ensemble. Ce système de visée, qui comprend une mire active MAM, présente au sujet l'image d'un point lumineux s'écartant périodiquement de l'axe de visée recherché. Le patient est alors invité à suivre tous les mouvements de cette image. Chaque fois que l'image revient sur l'axe, et après un temps de latence ajustable, une série de mesures interférométriques est réalisée. Le déplacement périodique du regard permet d'obtenir du patient une meilleure capacité de fixation quand il vise l'axe recherché. L'amplitude et la fréquence sont adaptables au sujet et aux mesures entreprises. Pour des raisons de commodité, la mire peut être réalisée avec un simple ordinateur de bureau sur lequel un point lumineux est affiché et déplacé. La mire active MAM, l'optique adaptative, la source S et la prise d'image sont synchronisées.

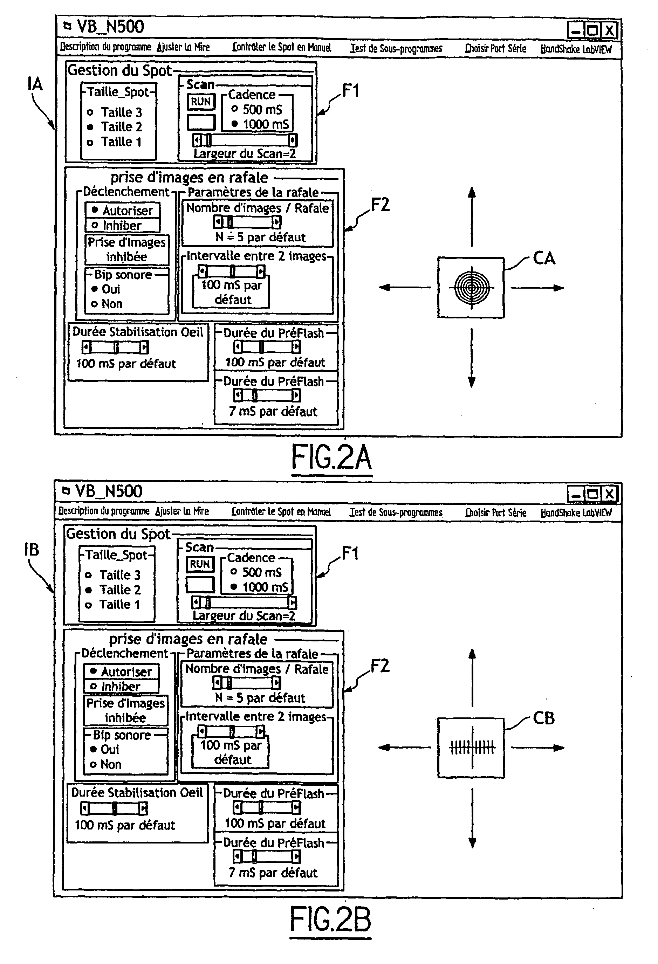

La mire active peut être réalisée sur l'écran d'un ordinateur ou d'un moniteur connecté à un système de commande (non représenté) du dispositif de visée, comme l'illustrent les figures 2A et 2B. Dans ce mode de réalisation, une interface graphique utilisateur IA ou IB comprend par exemple une première fenêtre F1 de gestion d'un spot, une seconde fenêtre F2 de prise d'image en rafale, et une cible mobile CA ou CB sur une zone de l'écran. Cette cible mobile peut être réalisée par exemple sous la forme d'une cible de représentation conventionnelle constituée d'un ensemble de cercles concentriques et d'une croix de visée au centre de ces cercles (Figure 2A), ou bien encore sous la forme d'un curseur gradué et d'une croix de visée superposée (Figure 2B)

Dans l'exemple illustré en figure 3, le système est agencé pour que la cible de la mire active MAM soit visible par les deux yeux OD1 et OG1 du sujet à examiner. Une visée avec les deux yeux peut en effet permettre d'améliorer les performances de fixations ou de stabilité, et faciliter l'examen. Dans cet exemple, l'image de la mire est introduite dans le trajet optique entre la source de référence SLD et l'oeil examiné par une séparatrice BST3.

Cette séparatrice peut être choisie dichroïque de manière à réfléchir 50% de toute la lumière venant de la mire MAM vers l'oeil examiné OEX, et transmettre les 50% restant vers l'autre oeil OV1 ou OV2 pour permettre une visée des deux yeux. La séparatrice dichroïque BST3 transmet alors toute la lumière de la source de référence SLD vers l'oeil examiné OEX, en profitant d'une différence de spectre entre la source de référence SLD (830 nm) et la mire MAM (800 nm). Une lame séparatrice 50/50 totalement neutre spectralement convient également, mais 50% de la lumière de la SLD est alors envoyé vers l'oeil qui n'est pas étudié. Un filtre peut permettre d'éliminer cette image si elle est jugée gênante par le sujet.

De façon à pouvoir examiner n'importe lequel des deux yeux tout en assurant une visée des deux yeux, le système présente un emplacement central d'examen OEX, ainsi que deux emplacements de visée OV1 et OV2 répartis des deux côtés de cet emplacement d'examen OEX.

Lorsque l'oeil gauche est à l'emplacement central pour être examiné, l'oeil droit reçoit l'image de la mire MAM dans son emplacement de visée OV1 par des moyens de renvoi escamotables, par exemples deux miroirs MT1 et MT2. Lorsque c'est l'oeil droit qui est à l'emplacement d'examen OEX, les moyens de renvoi peuvent être escamotés ou annulés et l'image de la mire MAM parvient à l'oeil gauche dans son emplacement de visée OV2.

Ainsi qu'illustré en figure 3, le système peut également comprendre, ou collaborer avec, des moyens de suivi IRIS des mouvements de l'oeil à examiner, collaborant avec le dispositif de tomographie. Il peut s'agir par exemple d'une caméra avec reconnaissance d'image réalisant un suivi ou « tracking », par exemple de la rétine ou de pupille ou des bords de l'iris, de façon à détecter et évaluer les mouvements de l'oeil.

La connaissance des mouvements de l'oeil permet alors au système de s'adapter aux déplacements de la zone à examiner, par exemple en coordonnant et les réglages et les prises de vue avec les différentes positions détectées ou prévues de cette zone à examiner, ou en permettant une optimisation spatiale et/ou temporelle de l'optique adaptive. Il est possible par exemple de profiter des périodes naturelles de stabilisation de la pupille ou de la rétine pour réaliser tout ou partie des réglages ou des mesures souhaités.

L'image de l'oeil examiné parvient aux moyens de suivi de l'oeil IRIS par une séparatrice BST2 insérée dans le trajet optique, par exemple entre l'oeil et la source de référence SLD. De façon avantageuse, par exemple pour ne pas gêner le sujet, cette séparatrice BST2 est dichroïque et le suivi des mouvements de l'oeil se fait en lumière non visible, par exemple infrarouge.

Les moyens de suivi IRIS peuvent comprendre par exemple un dispositif de mesure des déplacements oculaires, comme ceux développés par la société Métrovision.

L'invention peut en particulier être mise en oeuvre pour réaliser ou compléter un dispositif d'imagerie rétinienne, ou de topographie cornéenne, ou de mesure d'un film de larmes.

Bien sûr, l'invention n'est pas limitée aux exemples qui viennent d'être décrits et de nombreux aménagements peuvent être apportés à ces exemples sans sortir du cadre de l'invention.

## Revendications

1. Dispositif de visée (MAM) pour équiper un système d'examen de l'oeil d'un sujet, comprenant au moins une cible mobile (CA, CB) présentant une forme programmable ou une trajectoire programmable, ladite cible étant affichée sur des moyens de visualisation et visible d'au moins un oeil dudit sujet, pendant la durée de l'examen, ledit dispositif comprenant des moyens pour déplacer la ou les cible(s) de manière à alterner des intervalles de fixation sur une position donnée avec des intervalles dits de repos sur une ou plusieurs autres positions, ledit dispositif étant **caractérisé en ce qu'**il comprend en outre des moyens pour ajuster la durée des intervalles de fixation.

2. Dispositif selon la revendication 1, **caractérisé en ce qu'**il comprend en outre des moyens pour ajuster la diversité des positions de repos.

3. Dispositif selon l'une des revendications 1 ou 2, **caractérisé en ce qu'**il comprend en outre des moyens pour ajuster la durée des positions de repos.

4. Dispositif selon l'une des revendications 1 à 3, **caractérisé en ce qu'**il comprend en outre des moyens pour commander un mouvement continu d'une cible mobile.

5. Procédé de visée pour un examen de l'oeil d'un sujet, mis en oeuvre dans un dispositif selon l'une des revendications précédentes, comprenant un affichage sur des moyens de visualisation, pendant la durée de l'examen, d'au moins une cible mobile (CA, CB) présentant une forme programmable ou une trajectoire programmables et visible d'au moins un oeil dudit sujet, ledit procédé comprenant un déplacement de la ou des cible(s) de manière à alterner des intervalles de fixation sur une position donnée avec des intervalles dits de repos sur une ou plusieurs autres positions, ledit procédé-étant **caractérisé en ce qu'**il comprend en outre un ajustement de la durée des intervalles de fixation.

6. Procédé selon la revendication 5, caractérisé en qu'il comprend en outre un ajustement de la diversité des positions de repos.

7. Procédé de visée pour un examen de l'oeil d'un sujet, mis en oeuvre dans un dispositif selon l'une des revendications précédentes, comprenant un affichage sur des moyens de visualisation, pendant la durée de l'examen, d'au moins une cible mobile (CA, CB) présentant une forme programmable ou une trajectoire programmable et visible d'au moins un oeil dudit sujet, ledit procédé étant **caractérisé en ce qu'**il comprend en outre une commande d'un mouvement continu d'une cible mobile.

8. Procédé selon l'une des revendications 5 à 7, **caractérisé en ce qu'**il comprend en outre un suivi des mouvements de l'oeil à examiner.

9. Procédé selon la revendication 8, **caractérisé en ce que** le suivi des mouvements de l'oeil à examiner se fait par imagerie utilisant un spectre non visible.

10. Système d'examen de l'oeil par tomographie in vivo, comprenant :
- un interféromètre de Michelson, réalisant un montage de tomographie optique cohérente OCT plein champ,
- des moyens d'optique adaptative, disposés entre l'interféromètre et un oeil à examiner, réalisant une correction des fronts d'onde en provenance de l'oeil maïs aussi à destination de l'oeil et
- des moyens de détection, disposé en aval de l'interféromètre, permettant de réaliser la mesure interférométrique selon le principe de l'OCT plein champ sans modulation ni détection synchrone, **caractérisé en ce qu'**il comprend en outre un dispositif de visée comprenant au moins une cible mobile présentant une forme programmable ou une trajectoire programmable, ladite cible étant affichée sur des moyens de visualisation et visible d'au moins un des yeux dudit patient, pendant la durée de l'examen.

11. Système selon la revendications 10 , **caractérisé en ce qu'**il comprend des moyens (IRIS) de suivi des mouvements de l'oeil à examiner (OEX), collaborant avec le dispositif de tomographie.

12. Système selon l'une des revendications 10 ou 11, **caractérisé en ce qu'**il comprend des moyens pour faire parvenir l'image de la cible aux deux yeux (OV1, OEX) du sujet à examiner.

13. Système selon l'une des revendications 10 à 12, **caractérisé en ce qu'**il comprend des moyens pour faire parvenir l'image de la cible à l'oeil non examiné du sujet sélectivement d'un côté (OV1) ou de l'autre (OV2) de l'oeil examiné (OEX).

## Patentansprüche

1. Visiervorrichtung (MAM) zur Ausstattung eines Untersuchungssystems des Auges eines Subjekts, mindestens ein bewegliches Ziel (CA, CB) umfassend, das eine programmierbare Form oder einen programmierbaren Bewegungspfad aufweist, wobei jenes Ziel während der Dauer der Untersuchung auf Visualisierungseinrichtungen angezeigt wird und durch mindestens ein Auge jenes Subjekts sichtbar ist, wobei jene Vorrichtung Einrichtungen enthält, um das Ziel bzw. die Ziele so zu bewegen, dass Fixationsintervalle auf einer bestimmten Position mit sogenannten Ruheintervallen auf einer oder mehreren weiteren Positionen abwechseln, wobei die Vorrichtung **dadurch gekennzeichnet ist, dass** sie außerdem Einrichtungen zur Justierung der Dauer der Fixationsintervalle umfasst.

2. Vorrichtung nach Anspruch 1, **dadurch gekennzeichnet, dass** sie außerdem Einrichtungen zur Justierung der Vielfalt der Ruhepositionen umfasst.

3. Vorrichtung nach einem der Ansprüche 1 oder 2, **dadurch gekennzeichnet, dass** sie außerdem Einrichtungen zur Justierung der Dauer der Ruhepositionen umfasst.

4. Vorrichtung nach einem der Ansprüche 1 bis 3, **dadurch gekennzeichnet, dass** sie außerdem Einrichtungen zur Steuerung einer durchgehenden Bewegung eines beweglichen Ziels umfasst.

5. Visierverfahren für eine Untersuchung des Auges eines Subjekts, eingesetzt in einer Vorrichtung nach einem der vorhergehenden Ansprüche, während der Dauer der Untersuchung eine Anzeige auf Visualisierungseinrichtungen wenigstens eines beweglichen Ziels (CA, CB) umfassend, das eine programmierbare Form oder einen programmierbaren Bewegungspfad aufweist und durch wenigstens ein Auge jenes Subjekts sichtbar ist, wobei jenes Verfahren eine Bewegung des Ziels bzw. der Ziele umfasst, sodass Fixationsintervalle auf einer bestimmten Position mit sogenannten Ruheintervallen auf einer oder mehreren weiteren Positionen abwechseln, wobei jenes Verfahren **dadurch gekennzeichnet ist, dass** es außerdem eine Justierung der Dauer der Fixationsintervalle umfasst.

6. Verfahren nach Anspruch 5, **dadurch gekennzeichnet, dass** es außerdem eine Justierung der Vielfalt der Ruhepositionen umfasst.

7. Visierverfahren für eine Untersuchung des Auges eines Subjekts, eingesetzt in einer Vorrichtung nach einem der vorhergehenden Ansprüche, während der Dauer der Untersuchung eine Anzeige auf Visualisierungseinrichtungen wenigstens eines beweglichen Ziels (CA, CB) umfassend, das eine programmierbare Form oder einen programmierbaren Bewegungspfad aufweist und durch wenigstens ein Auge dieses Subjekts sichtbar ist, wobei jenes Verfahren **dadurch gekennzeichnet ist, dass** es außerdem eine Steuerung einer durchgehenden Bewegung eines beweglichen Ziels umfasst.

8. Verfahren nach einem der Ansprüche 5 bis 7, **dadurch gekennzeichnet, dass** es außerdem ein Nachverfolgen der Bewegungen des zu untersuchenden Auges umfasst.

9. Verfahren nach Anspruch 8, **dadurch gekennzeichnet, dass** das Nachverfolgen der Bewegungen des zu untersuchenden Auges über Abbildung mittels eines nicht sichtbaren Spektrums erfolgt.

10. System zur Untersuchung des Auges mittels in-vivo Tomographie, umfassend:
- ein Michelson-Interferometer, das eine Vollfeld- optische Kohärenztomographie- (OCT) Abbildung erzeugt,
- Adaptiv-Optikeinrichtungen, zwischen dem Interferometer und einem zu untersuchenden Auge angeordnet, eine Korrektur der Wellenfronten vom Auge aus aber auch zum Auge hin ausführend und
- Erkennungseinrichtungen, dem Interferometer nachgelagert angeordnet, die Ausführung der interferometrischen Messung nach dem Verfahren der Vollfeld-OCT ohne Modulation oder Synchrondemodulation ermöglichend,
**dadurch gekennzeichnet, dass** es außerdem eine Visiervorrichtung mit mindestens einem beweglichen Ziel mit einer programmierbaren Form oder einem programmierbaren Bewegungspfad umfasst, wobei jenes Ziel auf Visualisierungseinrichtungen angezeigt wird und durch wenigstens eins der Augen jenes Patienten während der Dauer der Untersuchung sichtbar ist.

11. System nach Anspruch 10, **dadurch gekennzeichnet, dass** es Einrichtungen (IRIS) zum Nachverfolgen der Bewegungen des zu untersuchenden Auges (OEX) umfasst, die mit der Tomographie-Vorrichtung zusammen arbeiten.

12. System nach einem der Ansprüche 10 oder 11, **dadurch gekennzeichnet, dass** es Einrichtungen umfasst, um beiden Augen (OV1, OEX) des zu untersuchenden Subjekts die Abbildung des Ziels zu vermitteln.

13. System nach einem der Ansprüche 10 bis 12, **dadurch gekennzeichnet, dass** es Einrichtungen umfasst, um dem nicht untersuchten Auge des Subjekts wahlweise auf einer (OV1) oder auf der anderen Seite (OV2) des untersuchten Auges (OEX) die Abbildung des Ziels zu vermitteln.

## Claims

1. Sighting device for equipping a system for examining the eye of a subject, comprising at least one moving target (CA, CB) having a programmable shape or trajectory, said target being displayed on viewing means and visible by at least one eye of said subject during the examination period, said device comprising means for moving the target(s) so as to alternate fixation intervals on a given position with intervals termed rest on one or more other positions, said device being **characterized in that** it further comprises means for adjusting the duration of the fixation intervals.

2. Device according to claim 1, **characterized in that** it also comprises means for adjusting the diversity of the rest positions.

3. Device according to one of claims 1 or 2, **characterized in that** it also comprises means for adjusting the duration of the rest positions.

4. Device according to one of claims 1 to 3, **characterized in that** it also comprises means for controlling a continuous movement of a moving target.

5. Sighting method for an examination of a subject's eye, implemented in a device according to one of the preceding claims, comprising a display on viewing means, during the examination period, of at least one moving target (CA, CB) having a programmable shape and trajectory and visible by at least one eye of said subject, said method comprising a movement of the target(s) so as to alternate fixation intervals on a given position with intervals termed rest on one or more other positions, said method being **characterized in that** it further comprises an adjustment of the duration of the fixation intervals.

6. Method according to claim 5, **characterized in that** it also comprises an adjustment of the diversity of the rest positions.

7. Sighting method for an examination of a subject's eye, implemented in a device according to one of the preceding claims, comprising a display on viewing means, during the examination period, of at least one moving target (CA, CB) having a programmable shape and trajectory and visible by at least one eye of said subject, said method being **characterized in that** it also comprises a control of a continuous movement of a moving target.

8. Method according to one of claims 5 to 7, **characterized in that** it also comprises a tracking of the movements of the eye to be examined.

9. Method according to claim 8, **characterized in that** the tracking of the movements of the eye to be examined is carried out by imaging using a non-visible spectrum.

10. System for examining the eye by *in vivo* tomography, comprising:
- a Michelson interferometer, producing a full field optical coherence tomography OCT set up,
- adaptive optical means, arranged between the interferometer and an eye to be examined, producing a correction of the wavefronts originating from the eye as well as those reaching the eye, and
- means of detection, arranged downstream of the interferometer, capable of carrying out the interferometric measurement according to the OCT principle without synchronous modulation or detection,
**characterized in that** it also comprises a sighting device comprising at least one moving target, having a programmable shape and trajectory, said target being displayed on viewing means and visible from at least one of the eyes of said patient during the examination period.

11. System according to claim 10, **characterized in that** it comprises means (IRIS) of tracking movements of the eye to be examined (OEX), collaborating with the tomography device.

12. System according to one of claims 10 or 11, **characterized in that** it comprises means to enable the image of the target to reach both eyes (OV1, OEX) of the subject to be examined.

13. System according to one of claims 10 to 12, **characterized in that** it comprises means to enable the image of the target to reach the unexamined eye of the subject selectively on one side (OV1) or on the other side (OV2) of the examined eye (OEX).
